# EUROPÄISCHE PATENTANMELDUNG

(11) **EP 0 890 567 A1**
(43) Veröffentlichungstag der Anmeldung: **13.01.1999**
(21) Anmeldenummer: 98110613.1
(22) Anmeldetag: 10.06.1998
(51) Int. Cl.: C07C 45/41

(54) **Verfahren zur Herstellung von Aldehyden**

(30) Priorität: 10.07.1997 DE 19729381
(71) Anmelder: BASF AKTIENGESELLSCHAFT, 67056 Ludwigshafen (DE)
(72) Erfinder: Fischer, Rolf, Dr., 69121 Heidelberg (DE); Liang, Shelue, Dr., 67105 Schifferstadt (DE); Schwab, Ekkehard, Dr., 67434 Neustadt (DE); Wulff-Döring, Joachim, Dr., 67227 Frankenthal (DE); Sterzel, Hans-Josef, Dr., 67125 Dannstadt-Schauernheim (DE)

(57) **Zusammenfassung**

Verfahren zur Herstellung von Aldehyden der Formel I

R-CHO I

aus Säuren der Formel II

R-COOH II

oder deren Estern mit Alkoholen mit 1 bis 10 C-Atomen, wobei R für einen gegebenenfalls substituierten und/oder gegebenenfalls Heteroatome enthaltenden aliphatischen, cycloaliphatischen, aromatischen oder araliphatischen Rest steht, durch selektive Hydrierung mit Wasserstoff in Gegenwart eines Metalloxid-Katalysators in der Gasphase, wobei man die Hydrierung bei Temperaturen von 150 bis 450°C über einem Träger-Katalysator durchführt, der als wesentliche Bestandteile ein zur selektiven Hydrierung von Carbonsäure oder Carbonsäureestern geeignetes Metalloxid auf SiC als Träger enthält.

## Beschreibung

Die Erfindung betrifft ein Verfahren zur Herstellung von Aldehyden durch Hydrierung von Carbonsäuren oder Estern in der Gasphase mit überschüssigem Wasserstoff über Trägerkatalysatoren, die als wesentliche Bestandteile ein zur selektiven Hydrierung zum Aldehyd geeignetes Metalloxid auf SiC als Träger enthalten.

Aus WO 95/33741 ist bekannt, Carbonsäuren und Carbonsäureester von C1-10-Alkoholen katalytisch bei Temperaturen über 200°C und Drücken von 0,1-20 bar mit Wasserstoff im Überschuß direkt zu Aldehyden zu hydrieren. Dafür eignen sich eine Reihe effektiver Katalysatoren wie Aluminiumoxid (US 3 935 265); Manganoxid (US 4 585 899, EP 290 096), Eisenoxid (EP 304 853), Ruthenium/Zinnoxid (EP 539 274), Vanadinoxid und Titandioxid (US 4 950 799, EP 414 065), Chrom-/Zirkonoxid (EP 150 961, 439 115), Chrom-/Titanoxid (EP 414 065), Yttrium-/Aluminiumoxid (US 4 328 373, EP 101 111) und Lanthan-/Zirkonoxid (DE-A-44 19 514; DE-A-44 43 704; DE-A-19 509 552).

Besonders gut bewährt hat sich der breit anwendbare Katalysator La₂O₃/ZrO₂ mit 0,1 bis 20 Gew.-% La₂O₃ auf monoklinem ZrO₂.

Bei Verwendung der vorgenannten Katalysatoren muß in der Regel das α-C-Atom des Edukts mindestens einfach substituiert sein, damit der Aldehyd durch selektive Hydrierung gebildet wird und nicht Ketone als unerwünschte Produkte entstehen.

Aus EP 439 115 und US 5 306 845 ist ferner bekannt, daß auch aus Carbonsäuren und Estern, aus deren α-C-Atom zwei Wasserstoffatome trägt, Aldehyde in guter Ausbeute entstehen, wenn man Cr₂O₃ als Vollkontakt verwendet. Für diese Reaktion sind hohe Temperaturen von bis zu 450°C erforderlich. Außerdem ist der C₂O₃-Vollkontakt mechanisch nicht stabil genug. Trägerkatalysatoren sind jedoch Vollkontakten vorzuziehen, da sehr viel geringere Mengen Aktivmetall erforderlich sind und die mechanische Stabilität von Trägerkattalysatoren besser ist.

Es bestand daher die Aufgabe, Trägerkatalysatoren für die selektive Hydrierung von Carbonsäuren oder deren Estern zu Aldehyden vorzuschlagen, die eine ausreichende Stabilität, gute Regenerierbarkeit, keine oder geringe Nebenproduktbildung und mindestens vergleichbare Selektivität wie die entsprechend Vollkontakte aufweisen.

Diese Aufgabe wurde erfindungsgemäß gelöst mit einem Verfahren zur Herstellung von Aldehyden der Formel I

R-CHO I

aus Säuren der Formel II

R-COOH II

oder deren Estern mit Alkoholen mit 1 bis 10 C-Atomen, wobei R für einen gegebenenfalls substituierten und/oder gegebenenfalls Heteroatome enthaltenden aliphatischen, cycloaliphatischen, aromatischen oder araliphatischen Rest steht, durch selektive Hydrierung mit Wasserstoff in Gegenwart eines Metalloxid-Katalysators in der Gasphase, wobei man die Hydrierung bei Temperaturen von 150 bis 450°C und Drücken von 0,1 bis 100 bar über einem Träger-Katalysator durchführt, der als wesentliche Bestandteile ein zur selektiven Hydrierung von Carbonsäure oder Carbonsäureestern geeignetes Metalloxid auf Siliciumcarbid (SiC) als Träger enthält.

Als für die selektive Hydrierung von Carbonsäuren oder deren Estern in den entsprechenden Aldehyden geeignete Metalloxide kommen z.B. Oxide der Metalle der Gruppen IVB, VB, VIB, VIIB und VIII des Periodensystems der Elemente, insbesondere Chrom, Mangan, Eisen, Zinn oder Gemische dieser Oxide in Betracht. Davon ist Chrom in Form von Cr₂O₃ bevorzugt.

Die Herstellung von SiC kann nach verschiedenen, aus der Literatur bekannten Methoden z.B. gemäß den Angaben in Gmelin Handbook of Inorganic Chemistry, Si, Supplement Vol. B3, S. 36ff., 8. Auflage, Berlin, 1986, oder vorzugsweise nach der Methode gemäß EP-A 0 313 480 erfolgen.

Die BET-Oberfläche des Trägers liegt bevorzugt bei Werten größer als 2 m²/g, besonders bevorzugt bei größer 5 m²/g. SiC kann sowohl in der hexagonalen als auch in der kubischen Form vorliegen, bevorzugt ist die kubische Form.

Die Aufbringung des Metalloxids auf das SiC erfolgt in an sich bekannter Weise, z.B. durch Tränken des Trägers mit einer Lösung des Salzes der genannten Elemente, Aufsprühen der Salzlösung oder Auffällen der Oxide und anschließender Calzinierung.

Das Gewichtsverhältnis des Metalloxids zum SiC-Träger beträgt in der Regel 1 zu 200 bis 1 zu 1, vorzugsweise 1 zu 100 bis 1 zu 5.

Der besonders bevorzugte Katalysator ist Cr₂O₃ auf SiC mit einem Gewichtsverhältnis von Metalloxid zu Träger 1 zu 100 bis 1 zu 5.

Der SiC-Träger wird in der Regel in Form von Strängen, Pellets oder Kugeln verwendet.

Als Ausgangsverbindungen können gegebenenfalls substituierte und/oder gegebenenfalls Heteroatome enthaltende aliphatische, cycloaliphatische, aromatische oder araliphatische Carbonsäuren oder ihre Ester verwendet werden. Beispiele hierfür sind n-Pentansäure, n-Hexansäure, Laurinsäure, Palmitinsäure, n-Hexansäuremethylester, Propionsäure, Cyclohexancarbonsäure, Benzoesäuremethylester, Benzoesäure, Tetrahydropyran-4-carbonsäuremethylester, Nicotinsäure oder Terephthalsäuredimethylester. Als Ester kommen solche von Alkoholen mit 1 bis 10 C-Atomen, bevorzugt mit 1 bis 4 und insbesondere mit 1 bis 3 C-Atomen in Betracht.

Besonders bevorzugt sind aliphatische Carbonsäuren und ihre Ester.

Die Aufarbeitung des Reaktionsprodukts zur Gewinnung des reinen Aldehyds erfolgt in an sich bekannter Weise durch Destillation. Während das Überreaktionsprodukt RCH₂OH ausgeschleust wird, kann die Esterfraktion RCOOCH₂R, die durch Umestern des Eduktesters RCOOR' mit dem Nebenprodukt-Alkohol RCH₂OH entsteht, in die Hydrierung zurückgeführt werden.

### Beispiele

### Beispiel 1 (Hydrierung von n-Hexansäure zu Hexanal)

a) Herstellung des Katalysators:
   210 g SiC (Pechiney, Splitt < 5 mm, Schneidhärte = 72 N) wurden in drei Schritten mit 185 ml einer wäßrigen Lösung, die 183,2 g Cr(NO₃)₃ x 9 H₂O (98 %ig) enthielt, getränkt. Zwischen den einzelnen Tränkschritten wurde der Katalysator jeweils 16 h bei 120°C getrocknet und 4 h bei 500°C calciniert.
   Der so hergestellte Katalysator enthielt 12 % Cr₂O₃, hatte eine BET-Oberfläche von 6,7 m²/g und das SiC lag kubisch vor.
b) Hydrierung:
   In einem Gasphasenreaktor wurden 80 ml Cr₂O₃ (12 %)/SiC (H-160)-Katalysator mit 20 ml Quarzringen als Verdampferstrecke überschichtet und anschließend mit Wasserstoff bei 400°C 2 Stunden aktiviert. Bei 400°C und Normaldruck wurden 8 g/h n-Hexansäure und 50 l/h Wasserstoff zudosiert. Der Austrag wurde in einer Vorlage kondensiert und die Ausbeuten gaschromatographisch durch Verwendung eines inneren Standards bestimmt. Nach 6 h Reaktionszeit wurde bei einem Umsatz von 81 % eine Hexanal-Selektivität von 92 % erhalten.

### Beispiel 2

Unter den im Beispiel 1 angegebenen Bedingungen wurde die Hydrierung am gleichen Katalysator bei 430°C durchgeführt. Hierbei betrug der Umsatz 100 % und die Hexanal-Selektivität 91 %.

### Beispiel 3 (Vergleich)

a) Herstellung des Katalysators:
   210 g ZrO₂ (5 x 3 mm Tabl.) wurden in zwei Schritten mit 110 ml einer wäßrigen Lösung, die 86,8 g Cr(NO₂)₃ x 9 H₂O (98 %ig) enthielt, getränkt. Zwischen den einzelnen Tränkschritten wurde der Katalysator jeweils 16 h bei 120°C getrocknet und 4 h bei 400°C calciniert.
   Der so hergestellte Katalysator enthielt 6 % Cr₂O₃ und hatte eine BET-Oberfläche von 103 m²/g.
b) Unter den im Beispiel 1 angegebenen Reaktionsbedingungen wurde die Hydrierung am Cr₂O₃ (6 %) ZrO₂-Katalysator bei 350°C durchgeführt. Bei einem Umsatz von 91 % wurde eine Hexanal-Selektivität von 53 % erhalten.

## Patentansprüche

1. Verfahren zur Herstellung von Aldehyden der Formel I
R-CHO I
aus Säuren der Formel II
R-COOH II
oder deren Estern mit Alkoholen mit 1 bis 10 C-Atomen, wobei R für einen gegebenenfalls substituierten und/oder gegebenenfalls Heteroatome enthaltenden aliphatischen, cycloaliphatischen, aromatischen oder araliphatischen Rest steht, durch selektive Hydrierung mit Wasserstoff in Gegenwart eines Metalloxid-Katalysators in der Gasphase dadurch gekennzeichnet, daß man die Hydrierung bei Temperaturen von 150 bis 450°C und Drücken von 0,1 bis 150 bar über einem Träger-Katalysator durchführt, der als wesentliche Bestandteile ein zur selektiven Hydrierung von Carbonsäure oder Carbonsäureestern geeignetes Metalloxid auf SiC als Träger enthält.

2. Verfahren gemäß Anspruch 1, dadurch gekennzeichnet, daß man Trägerkatalysatoren mit Metalloxiden des Chroms, Mangans, Eisen und/oder Zinns auf SiC verwendet.

3. Verfahren gemäß Anspruch 1, dadurch gekennzeichnet, daß man Trägerkatalysatoren verwendet, die als wesentliche Bestandteile Cr₂O₃ auf SiC enthalten.

4. Verfahren gemäß Anspruch 1, dadurch gekennzeichnet, daß SiC in der kubischen Form vorliegt.

5. Verfahren gemäß Anspruch 1, dadurch gekennzeichnet, daß die BET-Oberfläche des SiC-Trägers größer als 2 m²/g ist.

6. Verfahren gemäß Anspruch 1, dadurch gekennzeichnet, daß die BET-Oberfläche des SiC-Trägers größer als 5 m²/g ist.
